**Europäisches Patentamt**

**European Patent Office**

**Office européen des brevets**

(19)

(11) Publication number : **0 469 437 B1**

## (12) EUROPEAN PATENT SPECIFICATION

(45) Date of publication of patent specification :
**27.12.95 Bulletin 95/52**

(51) Int. Cl.⁶ : **G01N 37/00, G01N 33/00**

(21) Application number : **91112323.0**

(22) Date of filing : **23.07.91**

(54) **Method of and apparatus for preparing calibration gas**

Divisional application 95109386.3 filed on 23/07/91.

(30) Priority : **25.07.90 JP 194946/90**

(43) Date of publication of application :
**05.02.92 Bulletin 92/06**

(45) Publication of the grant of the patent :
**27.12.95 Bulletin 95/52**

(84) Designated Contracting States :
**DE GB**

(56) References cited :
**EP-A- 0 370 150
US-A- 3 776 023
SOVIET INVENTIONS ILLUSTRATED, Ch section, week 8544, December 1985 DERWENT PUBLICATIONS LTD., London, J 02 page 5
PATENT ABSTRACTS OF JAPAN, unexamined applications, P field, vol. 9, no. 314,December 10, 1985 THE PATENT OFFICE JAPANESE GOVERNMENT page 62 P 412**

(73) Proprietor : **HITACHI, LTD.**
**6, Kanda Surugadai 4-chome**
**Chiyoda-ku, Tokyo 101 (JP)**
Proprietor : **HITACHI TOKYO ELECTRONICS CO., LTD.**
**3-2, Fujihashi 3-chome**
**Ome-shi Tokyo (JP)**

(72) Inventor : **Irie, Takashi, Hitachi Daini Kyoshinryo**
**1-3, Higashikoigakubo-3-chome**
**Kokubunji-shi (JP)**
Inventor : **Mitsui, Yasuhiro**
**14-29, Nishifucho-4-chome**
**Fuchu-shi (JP)**
Inventor : **Mizokami, Kazuaki**
**Haitsu Namai 105,**
**2-26, Honcho-5-chome**
**Koganei-shi (JP)**

(74) Representative : **Patentanwälte Beetz - Timpe - Siegfried Schmitt-Fumian - Mayr**
**Steinsdorfstrasse 10**
**D-80538 München (DE)**

## Description

This invention relates to a method and apparatus for preparing a calibration gas containing a very low concentration of liquid sample vapor at a ppb ($10^{-9}$) level or below.

Conventionally, mixing of a liquid sample vapor with a gas at a known concentration has been performed using an apparatus described in JIS-K0226 (Japanese Industrial Standard) where the sample is water. In this method, water is provided in a glass vessel equipped with a diffusion tube, and a stream of water vapor is discharged into a stream of gas. The water concentration in the gas is determined by measuring the amount of vaporization of the water (that is, the amount of reduction of the water) in the vessel by the use of a microbalance.

According to the JIS, the calibration gas having a water concentration of 2 to 20 ppm can be obtained with an error of 1 ppm. With respect to an error below this value, calibration gases cannot be prepared with this method, and JIS does not define any method of preparing such gas mixtures.

According to a dilution method and apparatus disclosed in JP-A-60-41733, a gas sample of a standard concentration is diluted with a highly purified gas the flow rate of which is controlled by a capillary tube, so as to obtain a low-concentration gas. In this example, a one-stage dilution is used; however, by combining a plurality of such stages, the dilution ratio may be increased. With this method, it is possible to obtain a low-concentration gas for non-adhesive components; however, in this apparatus, valves are provided in the flow passage of a standard concentration gas. The valves have a large inner surface area, and therefore in the case of a gas containing a low concentration of a highly-adhesive component (e.g. water), the component is adsorbed on the inner surface, which results in the serious problem that low-concentration gases at ppb levels cannot be obtained. In addition to the large inner surface area, the valves have a complicated structure, and this involves the further problem that the cleaning of the valves cannot be effected easily, so that producing gas mixtures comprising water at a ppb level is not easy.

Analytical Chemistry 49, 1977, pages 270 to 275, discloses an example in which the measurement of water is carried out using Atmospheric Pressure Ionization Mass Spectrometry (APIMS). In this example, a water bottle with a small aperture is provided in the gas stream, and by changing the diameter of the small aperture of the water bottle, the water evaporation rate is controlled so as to prepare calibration gases of a ppm level, and this calibration gas is analyzed by APIMS to thereby obtain a calibration curve in the ppm region.

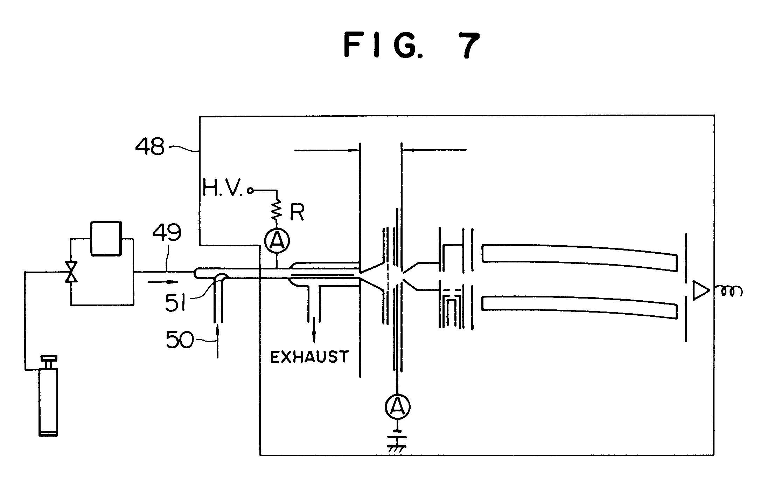

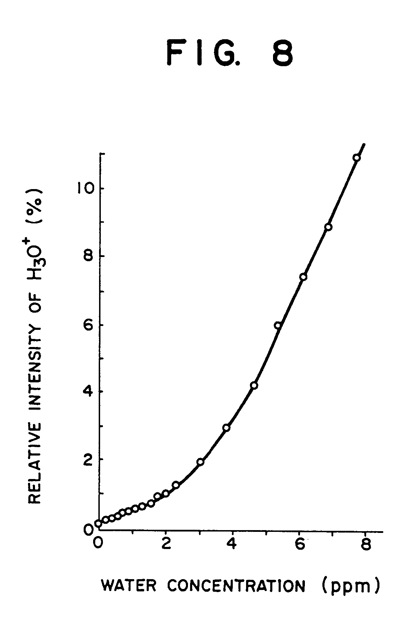

Fig. 7 shows a conventional apparatus, and Fig. 8 shows an example of a measured calibration curve. As shown in Fig. 8, even when only dry gas whose water concentration is zero is analyzed by APIMS, a water ion intensity corresponding to about 3 ppm of water is detected. Therefore, in the conventional apparatus shown in Fig. 7, considerable amounts of water are liberated at the sample gas introduction portion, so that calibration gases of a ppb level cannot be obtained at all. Therefore, a calibration curve of a ppb level cannot be obtained. It will be appreciated from this that in order to produce calibration gases of a ppb level and also to enable the measurement of the calibration curve in the ppb level region by APIMS, not only a suitable dilution method but also a reduction of the background water by appropriate structure and materials of the sample introduction system are indispensable.

One of the reasons why calibration curves can be obtained only at ppm levels in the conventional apparatus of Fig. 7 is that a high dilution ratio can not easily be obtained because the evaporation rate of water is controlled by changing the diameter of the aperture of the water bottle, and thus, the control of the gas production at a low concentration of a ppb level is difficult. However, if the problem is only with the dilution method, the standard gas containing a controlled concentration of the liquid sample vapor at a ppb level or below must be theoretically prepared by combining the water producing device of JIS-K0226 with a multistage dilution system (for example, a two-stage dilution system) constituted by a plurality of combined dilution systems according to JP-A-60-41733, that is, by preparing the calibration gas containing the liquid sample vapor of 2 to 20 ppm level and then by diluting it by the two-stage dilution system. However, with respect to such a liquid sample as water, (1) it takes a long time to establish an equilibrium between the adsorption and desorption of the sample relative to the inner surface of the tube because the effect of adsorption on the inner surface of the tube is great. As a result, the precision of the concentration is lowered, and the measurement time is prolonged; (2) since the background produced from the tube is large, the dilution ratio does not accurately reflect the concentration at a ppb level; (3) the coexisting components which quench the sample ions are produced to a considerable extent. Because of these problems, with respect to the two-stage gas dilution system, the area of the inner surface and the dead zone must be reduced by a compact construction thereof, and also the adsorption effects must be reduced by treating the inner surface of the tube and by heating the tube to elevated temperatures. In the above prior art, however, the gas inlet system is assembled with valves and flow controllers, or a tube not subjected to an inner surface treatment is used, and no consideration has been given to these problems. Therefore, using the apparatus shown in Fig. 7, it has been impossible to prepare or analyze calibration gases of a ppb level because the water background is high, as shown in Fig. 8.

It is the object of this invention to provide a method and an apparatus by which a calibration gas, containing a controlled, very low concentration of a highly-adhesive component, such as water, at a ppb level or below, can be rapidly prepared with a precise concentration.

The above object is achieved according to the independent claims. The dependent claims relate to prefered embodiments.

The method of the present invention for preparing a calibration gas comprises the steps of

- supplying a first zero gas from a first zero gas source with a controlled flow rate,
- supplying a sample gas from a sample gas source with a controlled flow rate,
- mixing the sample gas with the first zero gas in a mixing chamber thus forming a first standard gas containing the sample gas in a predetermined concentration,
- discarding a part of the first standard gas at a first branch with a controlled flow rate, and
- mixing the remaining first standard gas at a second branch downstream of the first branch with a second zero gas from a second zero gas source with a controlled flow rate to obtain a diluted, second standard gas;

it is characterized by

- supplying the sample gas by vaporizing a liquid sample as sample gas source with a controlled evaporation rate,
- discarding a part of the second standard gas at a third branch with a predetermined flow rate, and
- mixing the remaining diluted standard gas at a fourth branch downstream of the third branch with a third zero gas from a third zero gas source with a controlled flow rate, wherein the diluted standard gases are prevented from coming into contact with any parts such as valves and flow controllers, to obtain a calibration gas containing the sample gas in a very low concentration.

The apparatus of the present invention for preparing a calibration gas comprises

- a first and a second zero gas source for supplying a first zero gas and a second zero gas, comprising means for controlling the flow rates of the first or second zero gases respectively,
- a sample gas source for supplying a sample gas, comprising means for controlling the flow rate of the sample gas,
- a mixing chamber for mixing the sample gas with the first zero gas, thus forming a first standard gas, and

- a main tube connected with the mixing chamber and having
  (a) a first branch comprising controlling means for discarding a part of the first standard gas with a controlled flow rate,
  (b) a second branch provided downstream of the first branch, at which the remaining first standard gas is mixed with the second zero gas with a controlled flow rate for obtaining a diluted, second standard gas;

it is characterized in that

- the mixing chamber contains a sample vessel in which a liquid sample may be vaporized for supplying the sample gas,
- the means for controlling the flow rate of the sample gas are means for controlling the evaporation rate of the liquid sample from the sample vessel,
- the main tube is provided with
  (a) a third branch comprising controlling means for discarding a part of the diluted second standard gas with a predetermined flow rate, and
  (b) a fourth branch provided downstream of the third branch, at which the remaining diluted second standard gas is mixed with a third zero gas supplied from a third zero gas source comprising means for controlling the flow rate of the third zero gas, the main tube not being provided with any parts such as valves and flow controllers.

A method and an apparatus corresponding to the features of the preambles of the independent claims have been known from EP-A- 370 150.

US 3 776 023 discloses a portable and compact calibration system for gas analyzers which comprises a permeation tube containing a liquified gas, from which a sample gas permeates through a membrane into a stream of purified air as zero gas giving a standard gas with a precise concentration of the sample gas. The concentration of the sample gas, which is e.g. $SO_2$, $NO_2$, $Cl_2$ or a hydrocarbon, is changed by varying the pumping rate of the incoming zero gas, by adjusting the flow rate of the incoming zero gas directly before the permeation tube and by controlling the temperature of the permeation tube and of the incoming zero gas. Therefore the sample gas is diluted in only one step. The specification does not disclose a multi-step system for a precise dilution of sample gases to a ppb level.

In the present invention, a predetermined quantity of standard gas is produced from a gas source, and then a predetermined proportion of the gas is exhausted at least once, and then a predetermined amount of dilution gas is supplied at least once, thereby obtaining a calibration gas containing a very low concentration of liquid sample vapor. In the present invention, gas prepared beforehand, for example, a cylinder gas, can be used as the standard gas.

In order to achieve the above object, the structure and material of a device for preparing a standard gas containing a known concentration of a vaporized sample at a ppm level, as well as the structure and material of a two-stage gas dilution system, have been studied, and the following contrivances have been made. (1) Electropolished stainless steel tubes or glass tubes are used and connected together entirely by welding, so as to achieve a compact construction of the apparatus, thereby reducing the area of the inner surface and also minimizing the dead zone where the gas stream stagnates. (2) Any parts (such as valves and flow controllers which would be sources of background, and coexisting components which could quench sample ions are not comprised in the main tube. (3) The gas is controlled in pressure and flow rate by a gas regulator and a flow controller, and then the impurities are removed from the gas by an impurity removal device, and then the gas is introduced into the device for preparing the standard gas containing a known concentration of the vaporized sample at a ppm level or the two-stage gas dilution system. (4) The main tube of the two-stage gas dilution system is maintained at a temperature 20 to 50°C higher than the boiling point of the liquid sample so as to efficiently establish an equilibrium between the adsorption and desorption of the sample relative to the inner surface of the tube.

In the apparatus of the present invention, the calibration gas containing a very low concentration of the liquid sample at a ppb level is prepared according to the following procedure. First, the standard gas containing a high concentration (ppm level) of the vaporized sample is prepared in the following manner.

The gas is controlled in pressure by the gas regulator, and is controlled in flow rate by the flow controller, and then is introduced into a sample chamber. A sample bottle (vessel) which has a small aperture and contains the liquid sample is accommodated within the sample chamber. The vapor of the liquid sample is discharged into the stream of the gas through this aperture, and is mixed therewith. The upper surface of the sample bottle with the small aperture is a planar surface or a curved surface having no projection, and the small aperture is formed in this surface. In JIS-K0226, it is defined that a sample bottle should have a projection like a needle for vaporized sample diffusion, in which case the sample liquid often stagnates midway in this needle-like diffusion projection, so that the amount of vaporization is rendered unstable. For this reason, such a projection is eliminated in the present invention. Therefore, the sample liquid will not stagnate midway in the diffusion projection, and the standard gas containing a high concentration (ppm level) of the vaporized sample can be obtained in a stable manner.

By providing the impurity removal device (e.g. a molecular sieve trap) at the upstream side of the sample chamber, adhesive impurities, such as water and organic components, are removed from the gas. Therefore, the concentration precision of the standard gas containing the high concentration of the vaporized sample is not lowered, and also coexisting components which quench sample ions will not be introduced into the gas. The flow controller serves to keep constant the flow rate (L1 ($\ell$/min)) of the gas flowing through the sample chamber, and temperature control means provided outside the sample chamber serve to keep the temperature of the sample constant. Therefore, the high-concentration vaporized sample-containing standard gas which is always kept at a constant concentration and a constant flow rate is supplied to the two-stage gas dilution system.

For calibrating the concentration of the sample in the standard gas containing the high concentration of the vaporized sample, the amount of vaporization (i.e., the vaporized quantity) of the liquid sample in the sample bottle when the gas continues to flow at a constant rate for a long period of time is measured by a micro-balance.

This calibration method is the same as that described in JIS-K0226. More specifically, the concentration C (ppm) of the standard gas containing the high concentration of the vaporized sample is expressed by the following formula:

$$C = \frac{22.4 \times \dfrac{R}{M}}{L1 \times \dfrac{273}{273 + t} \times \dfrac{P}{760}},$$

where R ($\mu$g/min) represents the vaporized quantity of the liquid sample per unit time, M represents the molecular weight of the liquid sample, t (°C) represents the temperature, P (torr) represents the gas pressure, and L1 ($\ell$/min represents the gas flow rate.

If, for example, water is used as liquid sample, and the diameter of the aperture of the sample bottle is 1 mm, and when the gas is flowing at a flow rate of 1 $\ell$/min at 20°C and 760 torr (101325 Pa) for 10 h, the vaporized quantity is about 14 mg, and therefore the concentration of the standard gas containing the high concentration of the vaporized sample is about 30 ppm. In the case of using dioctyl phthalate (DOP) a liquid sample at 170°C, and for a diameter of the aperture of the sample bottle of 5 mm, and a gas flow rate of 0.3 $\ell$/min for 6 h, the vaporized quantity is about 2 mg, and therefore the concentration of the standard gas containing the high concentration of the vaporized sample is about 1.7 ppm.

The diameter of the aperture of the sample bottle is an important factor in determining the amount of vaporization of the sample into the gas, and needs to be changed depending on the vapor pressure of the sample. More specifically, the aperture diameter must be made small with respect to a sample having a high vapor pressure, and must be made large with respect to a sample having a low vapor pressure so as to keep the concentration of the sample in the standard gas (which contains the high concentration of the vaporized sample) to a level of 1 ppm to several tens of ppm. Referring to the reason for this, if the concentration is too high, the gas cannot be diluted from the maximum dilution ratio of the two-stage gas dilution system to a ppb level. On the other hand, if the concentration is too low, the vaporized quantity of the liquid sample is small, and even by the use of the microbalance, the precision of the weighing (and hence the precision of the calibration) is lowered because of weight variations caused by adsorption of water in the air on the sample bottle and the sample itself. In this case, in order to obtain a sufficient vaporized quantity, a long period of time is required for the calibration of the concentration. Thus, these disadvantages are encountered.

Actually, by changing the temperature of the sample by the temperature control means provided outside the sample chamber, the vapor pressure can be controlled, and therefore the sample concentration is not controlled only by the aperture diameter. However, as the set temperature deviates from the ordinary (room) temperature (around 20°C), the temperature control means cannot maintain a uniform gas and sample temperature, which would become complicated. Therefore, also the control of the aperture diameter is important. When water is used as the liquid sample, a standard gas having a sample concentration of about 1 to about 30 ppm can be obtained by setting the aperture diameter to 0.1 to 1 mm. In the case of dioctyl phthalate, a standard gas having a sample concentration of about 1 to about 10 ppm can be obtained with an aperture diameter of 5 mm.

Reference is now made to a method in which the high-concentration vaporized sample-containing standard gas prepared according to the above procedure is diluted by the two-stage gas dilution system to prepare the calibration gas containing a very low concentration of liquid sample vapor at a ppb level or below.

The standard gas containing the high concentration of the vaporized sample is introduced into the main tube, and then part of this standard gas is exhausted at a first branch (flow rate: L2 ($\ell$/min)). The flow rate of the exhausted gas is controlled by a flow controller provided at the first branch. The reason for the exhausting of the gas at the first branch is that assuming that any part of the gas is not exhausted (L2 = 0), in order to obtain a high dilution ratio (for example, 100 times), L3 must be about 100 times as large as L1. Usually, in order to achieve stability of the concentration of the standard gas containing the high concentration of the vaporized sample, L1 must be about 1 $\ell$/min, and therefore L3 must be about 100 $\ell$/min. To use such a large quantity of gas is uneconomical. Actually, if L1 and L2 are 1 $\ell$/min and 0.99 $\ell$/min, respectively, then L3 = 1 $\ell$/min is only required for obtaining a 100 times dilution ratio. This is economical. The remaining flow rate (L1 - L2) of the standard gas containing the high concentration of vaporized sample is diluted by gas (flow rate: L3 ($\ell$/min)) introduced from a second branch (first-stage dilution). The gas introduced from the second branch is controlled in pressure and flow rate by a gas regulator and a flow controller, and then impurities are removed from this gas by an impurity removal device. Therefore, the background vapor and coexisting components which quench sample ions are not introduced into the gas. With this first-stage dilution, there is obtained a gas the flow rate of which is represented by L1 - L2 + L3 ($\ell$/min), and the concentration of the sample in the gas is represented by the following formula:

$$C \times \frac{L1 - L2}{L1 - L2 + L3} \text{ (ppm)},$$

where C represents the concentration of the sample in the standard gas containing the high concentration of vaporized sample.

Part of the gas (flow rate: L1 - L2 + L3) obtained by the first-stage dilution is exhausted from a third branch (flow rate: L4 ($\ell$/min)). The gas is exhausted from the third branch for the same reason described above for the exhausting of the gas at the first branch.

The gas of the remaining flow rate (L1 - L2 + L3 - L4) is again diluted by gas (flow rate: L5 ($\ell$/min)) introduced from a fourth branch (second-stage dilution). Like the gas introduced from the second branch, the gas introduced from the fourth branch is controlled in pressure and flow rate by a gas regulator and a flow controller, and then impurities are removed from this gas by an impurity removal device. With this two-stage dilution, there is obtained the calibration gas having a very low concentration of vaporized sample where the flow rate is represented by L1 - L2 + L3 - L4 + L5, and the concentration is represented by C [(L1 - L2)/(L1 - L2 + L3)][(L1 - L2 + L3 - L4)/(L1 - L2 + L3 - L4 + L5)]. Here, C represents the concentration of the sample in the standard gas containing the high concentration of the vaporized sample.

In the above procedure, the gases, introduced respectively from the device for preparing the standard gas containing a known concentration of the vaporized sample and the second and fourth branches of the two-stage gas dilution system, are of the same kind.

If mass flow controllers are used as flow controllers for controlling the flow rates L1 to L5, the mass flow rates can be directly measured, and therefore errors in the measurement of L1 to L5 due to variations of the gas volume caused by the gas pressure and the gas temperature can be avoided, thereby enabling a highly precise dilution.

In order that in the very low concentration range of a ppb level, the actual concentration can immediately correspond correctly to the concentrations determined by the above flow rate controls (L2 to L5), it is important to reduce the generation of the background at the main tube and also to rapidly establish an equilibrium between the adsorption and desorption of the sample relative to the inner surface of the tube. In the present invention, the main tube is constituted by welding electropolished stainless steel tubes or glass tubes, and the short straight sections of the main tube are connected together so as to minimize the area of the inner surface thereof. Those parts (such as joints, valves and flow controllers) which would form dead zones and sources of impurities are eliminated from the main tube. Further, by the use of the temperature control means, the temperature of the main tube is maintained at a temperature 20 to 50°C higher than the boiling temperature of the liquid sample. With this arrangement, a rapid establishment of equilibrium between adsorption and desorption as well as a reduction of the background can be achieved.

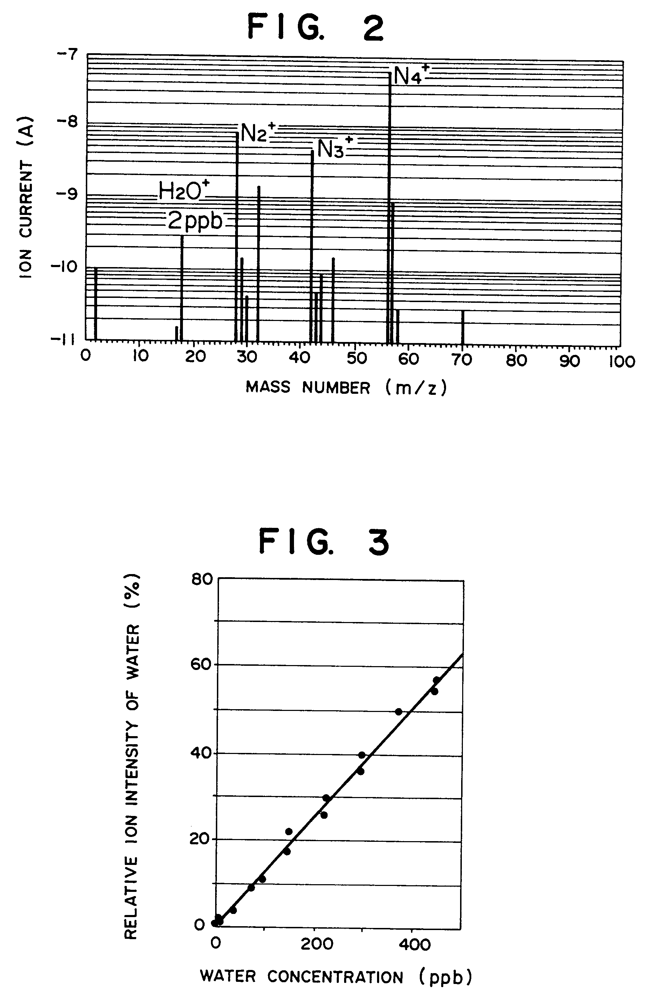

As one example, using the present invention, the background for the water in nitrogen gas can be reduced to 2 ppb by shortening the main tube to a length of 30 cm. This has been confirmed by APIMS, and results thereof are shown in Fig. 2. At this time, the time required for establishing equilibrium between adsorption and desorption was within about several tens of seconds.

As described above, by controlling the flow rates L2 to L5 at the two-stage dilution portion, the calibration gas containing a controlled known concentration of the liquid sample vapor in a very low concentration range of a ppb level can be prepared.

BRIEF DESCRIPTION OF THE DRAWINGS

Fig. 1 is a schematic view of one preferred embodiment of an apparatus of the present invention;

Fig. 2 is a diagram showing data of the background level of water produced from the apparatus of Fig. 1 which data are measured by APIMS;

Fig. 3 is a graph showing results of measurement of a calibration curve by APIMS for water, using calibration nitrogen gas containing a very low concentration of water which gas was prepared by the apparatus of Fig. 1;

Fig. 4 is a schematic view of another embodiment of an apparatus of the invention employed in facilities such as clean rooms, in which a line gas is used;

Fig. 5 is a schematic view of a further embodiment of an apparatus of the invention in which an analysis device such as a gas chromatograph is used for calibrating the concentration of a standard gas containing a high concentration of a sample;

Fig. 6 is a schematic view of a further embodiment of an apparatus of the invention in which the invention is applied to the calibration of an analysis device (e.g. an APIMS spectrometer or a dewpoint hygrometer) for analyzing a very low concentration of impurities in gas, and a calibration curve is automatically prepared;

Fig. 7 is a schematic view of a conventional apparatus for measuring a calibration curve of water by APIMS; and

Fig. 8 is a graph showing a calibration curve of water in oxygen gas which calibration curve was measured by the use of the apparatus of Fig. 7.

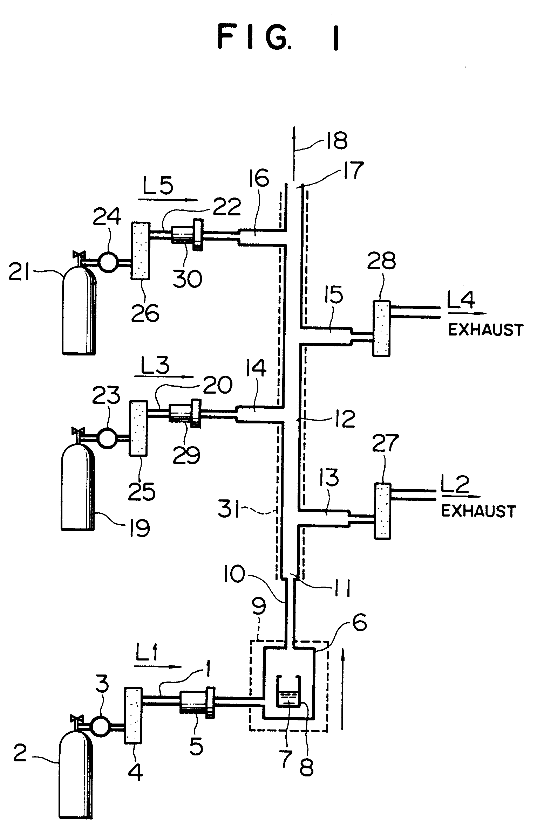

A preferred embodiment of the present invention will now be described with reference to Fig. 1.

The apparatus of the invention comprises a device for preparing standard gas containing a known concentration of a vaporized sample at ppm level (this standard gas will be hereinafter referred to as "high-concentration standard gas"), and a two-stage gas dilution system. In the device for preparing the high-concentration standard gas, first zero gas 1 is fed from a gas cylinder 2, and is controlled in pressure and flow rate by a gas regulator 3 and a flow controller 4. Adhesive impurities, such as water and organic components, are removed from the gas 1 by an impurity removal device 5, such as a molecular sieve trap, and then the gas 1 is introduced into a mixing chamber 6 serving as sample vapor-generating device. The impurities to be removed by the impurity removal device 5 include those originally contained in the gas 1 and those components originating from the gas regulator and the flow controller. In the case where the gas regulator and the flow controller cannot be sufficiently cleaned by high-temperature heating and the passing of gas therethrough, the use of the impurity removal device 5 is indispensable for the preparation of the calibration gas containing a sample whose concentration is at a ppb level or below, because such impurities adversely affect the preparation of this calibration gas.

A sample vessel 8 containing a liquid sample 7 is accommodated within the mixing chamber 6. The sample vessel 8 has an aperture with a diameter of 0.1 to 5 mm, and the vapor of the sample 7 is discharged into a stream of the gas 1 through this aperture. The whole mixing chamber 6 is maintained at a predetermined temperature by temperature control means 9, and the pressure of the vapor of the liquid sample 7 (and hence the amount of discharge of the sample vapor into the stream of the gas 1) is kept constant. The concentration of the sample 7 in the gas 1 (hereinafter referred to as "gas 10" (first standard gas)) passed past the mixing chamber 6 is calibrated by measuring the amount of vaporization of the sample 7 by a microbalance when the gas 1 continues to flow at a constant rate for a long period of time (about 10 h).

As one example, water is used as the sample 7, and the temperature of the mixing chamber 6 is maintained at 20°C, and the flow rate of the gas 1 is maintained at 1 $\ell$/min. In this case, when the diameter of the aperture of the sample vessel 8 is 1 mm, the water concentration of the gas 10 is about 30 ppm. As another example, the dioctyl phthalate (DOP) is used as the sample 7, and the temperature of the mixing chamber 6 is maintained at 170°C, and the flow rate of the gas 1 is maintained at 0.3 $\ell$/min. In this case, when the diameter of the aperture of the sample bottle 8 is 5 mm, the DOP concentration of the gas 10 is about 2 ppm.

The gas 10 thus containing a predetermined concentration of the sample 7 is introduced into a main tube (vaporized sample supply passage) 12 of the two-stage gas dilution system via a gas inlet 11.

The two-stage gas dilution system comprises the main tube 12, four branches 13, 14, 15 and 16, the gas inlet 11, and a gas outlet 17. In this two-stage gas dilution system, the gas 10 containing the predetermined concentration of the sample 7 is diluted according to the following procedure to be converted into a calibration gas 18 containing a very low concentration (ppb level) of the vaporized sample 7 (This calibration gas 18 will hereinafter be referred to as "the very low-concentration calibration gas 18").

The gas 10 of a constant flow rate L1 containing the predetermined concentration of the sample 7 is introduced into the main tube 12 via the gas inlet 11, and part (flow rate: L2) of the gas 10 is first exhausted at the first branch 13. This flow rate control is effected by a flow controller 27. Then, the gas 10 is diluted at the second branch 14 by gas 20 (flow rate: L3) supplied from a gas cylinder 19 (first-stage dilution). Part (flow rate: L4) of this first-stage diluted gas is exhausted at the third branch 15. This flow rate control is effected by a flow controller 28. The diluted gas is further diluted at the fourth branch 16 by gas 22 (flow rate: L5) supplied from a gas cylinder 21 (second-stage dilution) to provide the very low-concentration calibration gas 18 which is discharged from the gas outlet 17. The zero gases 20 and 22 are of the same kind as that of the zero gas 1, and are respectively controlled in pressure and flow rate by gas regulators 23 and 24 and flow controllers 25 and 26. For the same reasons described above for the gas 1, adhesive impurities are sufficiently removed from the gases 20 and 22 by impurity removal devices 29 and 30.

Incidentally, if the gases 1, 20 and 22 have a sufficiently high purity, and if the amounts of the impurities released from the gas regulators 3, 23 and 24 and the flow controllers 4, 25 and 26 are sufficiently small, the impurity removal devices 5, 29 and 30 may be omitted.

The concentration of the sample 7 in the very low-concentration calibration gas 18 prepared according to the above procedure is represented by C [(L1 - L2)/(L1 - L2 + L3)][(L1 - L2 + L3 - L4)/(L1 - L2 + L3 - L4 + L5)]. Here, C represents the concentration of the sample in the standard gas 10 of the constant flow rate L1. By changing the flow rates L2, L3, L4 and L5 respectively by the flow controllers 27, 25, 28 and 26, the calibration gas 18 containing a controlled, very low concentration of the sample can be obtained.

In order to obtain the calibration gas 18 containing the very low concentration of the sample at 1 ppb level or below, it is important that the generation of the impurities at the two-stage gas dilution portion should be reduced. Further, in order to improve the concentration precision and also to cause the concentration of the very low-concentration calibration gas 18 to be changed in a real time manner in response to the change of the set value of the concentration by the control of the flow controllers 27, 25, 28 and 26, it is important that the equilibrium between the adsorption and desorption of the sample relative to the inner surface of the tube should be established rapidly. To achieve this, the two-stage gas dilution system is constituted by tubes subjected to an inner surface treatment, or glass tubes, connected together entirely by welding so as to provide a compact construction of the two-stage dilution portion and also to reduce the dead zones, and further the temperature of the tube is maintained by heating means 31 at a temperature 20 to 50°C higher than the boiling temperature of the sample 7. Further, the flow control devices, such as the valves and the flow controllers, which would form sources of impurities are all mounted on the tubes of the branches, and are not mounted on the main tube 12 through which the standard or calibration gas flows.

The apparatus of Fig. 1 was actually prepared, and high-purity nitrogen gas was used as the gases 1, 20 an 22, and the background was measured using APIMS. Data of these measurements are shown in Fig. 2. The two-stage gas dilution system was made with tubes of SUS316L-EP, and the main tube 12 was shortened to 30 cm, thereby reducing the background of the water to 2 ppb.

A calibration nitrogen gas containing a very low concentration of water at a ppb level was prepared using the apparatus of Fig. 1, and the relation between the ion intensity and the water concentration (calibration curve) was measured. Results of these measurements are shown in Fig. 3. The calibration curve is satisfactorily linear, and this indicates that the dilution was carried out properly. Further, the time required for establishing the equilibrium between the adsorption and desorption was within several tens of seconds. Therefore, a real time analysis could be made.

In this embodiment, by combining the device for preparing the high-concentration standard gas with the two-stage gas dilution portion with respect to which the temperature control and the study of the structure and material have been made, there can be prepared calibration gases containing the vapor of the liquid sample in a wide concentration range from a ppb level to a ppm level.

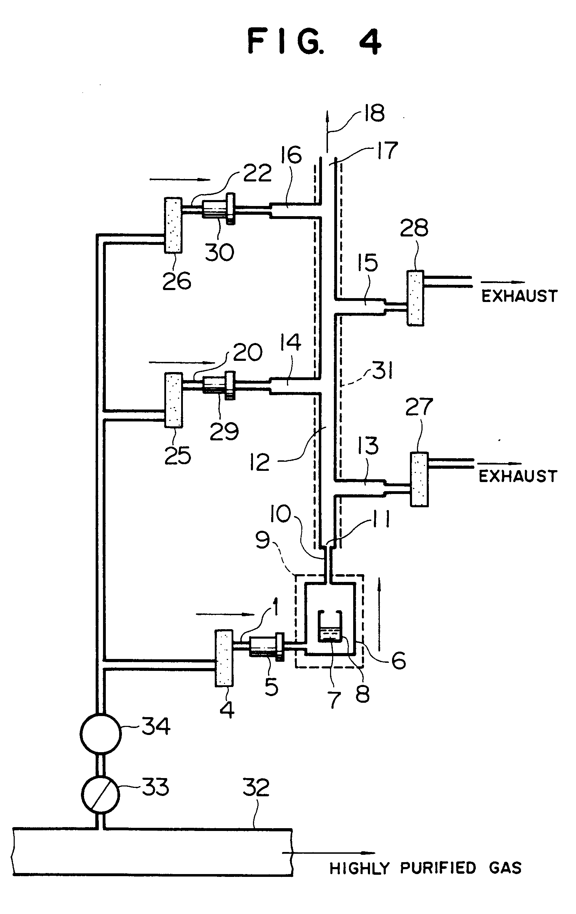

Fig. 4 shows an installation in which a highly-purified gas used in clean rooms is supplied in a branched manner from a highly-purified gas supplying system directly to an apparatus similar to the apparatus shown in Fig. 1. Line gas 32 is taken from the branch via a valve 33, and is controlled in pressure and flow rate by a gas regulator 34 and flow controllers 4, 25 and 26. The method of preparing a high-concentration standard gas 10 and a two-stage gas dilution method are the same as those in Fig. 1.

In this embodiment, the gas used in clean rooms can be directly introduced into the apparatus, and therefore additional facilities for installing the gas cylinders are unnecessary, and advantageously the gas can be supplied for a long period of time, and time and labor for the exchange of the gas cylinders are not required. In this embodiment, instead of the line gas, a cylinder gas may be used, in which case only one gas cylinder is needed, which is more convenient as compared with the apparatus of Fig. 1.

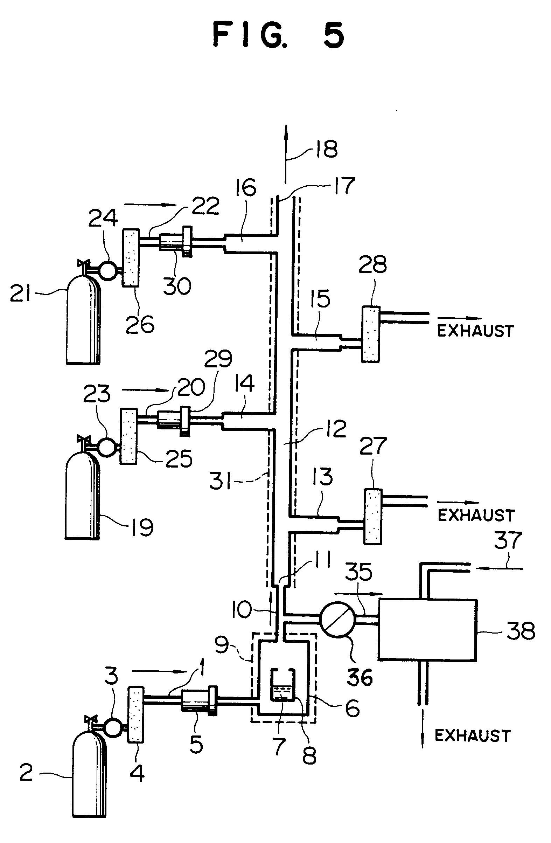

Fig. 5 illustrates a method in which the calibration of the concentration of the high-concentration standard gas 10, obtained by passing the gas 1 through the high-concentration standard gas preparing device of Fig. 1, is carried out not by a microbalance, but by an analysis device 38 such as a gas chromatograph. After the gas 10 is branched, this branch gas 35 is introduced into the analysis device 38 for a predetermined period of time by opening and closing a valve 36. The thus introduced gas is diluted with carrier gas 37, and is subjected to chromatography. The analysis device 38 has before-hand effected the concentration calibration with respect to the sample 7, and therefore the concentration can be known immediately from the data obtained.

Referring to the advantages of this embodiment, it does not take a long period of time to calibrate the concentration of the high-concentration standard gas 10 (the time required for this calibration is about 10 h), and the sample vessel 8 does not need to be taken out from the mixing chamber 6 each time the calibration is effected, and the concentration of the high-concentration standard gas 10 can be always monitored continuously.

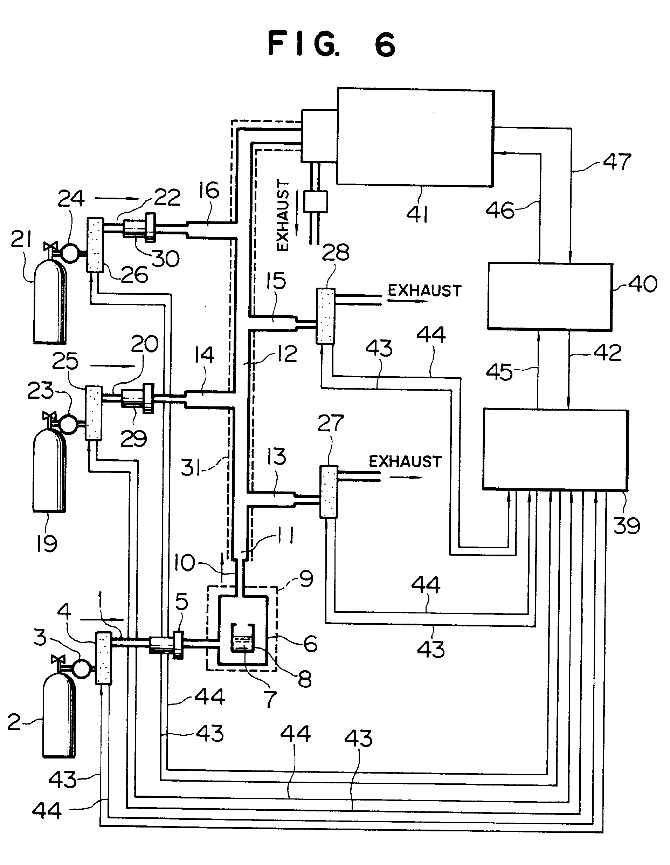

Fig. 6 shows a further embodiment of the invention in which using the apparatus shown in Fig. 1, the calibration of an analysis device (e.g. APIMS spectrometer) for analyzing a very low concentration of impurities in gas is carried out.

The apparatus of Fig. 1 is connected directly to the analysis device 41 for analyzing a very low concentration of impurities in gas, and the very low-concentration calibration gas 18 is introduced into the analysis device 41, so that the spectra can be observed. By controlling the flow controllers 25, 26, 27 and 28, the concentration is set to a desired value, and the ion intensity of the sample is measured with respect to each of the concentrations, and the calibration curve representative of the relation between the concentration and the ion intensity can be measured.

In this embodiment, the flow controllers 4, 25, 26, 27 and 28 are controlled by a flow controller-controlling unit 39 and a computer system 40. More specifically, when the set concentrations are input into the computer system 40, the set values of the flow rates L1 to L5 (here, L1 is constant) are automatically calculated, and a flow rate-setting data signal 42 is fed to the controlling unit 39. The controlling unit 39 sends set flow rate signals 43 to the respective flow controllers, and reads flow rate data signals 44, fed respectively from the flow controllers, so as to confirm that the flow rates have been properly set respectively in the flow controllers. Thereafter, the controlling unit 39 sends a confirmation signal 45 to the computer system 40. Upon receipt of this confirmation signal 45, the computer system 40 sends a data input-starting signal 46 to the analysis device 41, and then receives a data signal 47 from the analysis device 41 and processes this data signal to prepare the spectra.

In this embodiment, there is a great advantage that the calibration curve of the analysis device for analyzing the very low concentration of impurities in the gas containing the liquid sample 7 can be automatically produced. And besides, the computer system 40 can be used not only to control the flow controllers but also to control the analysis device for analyzing the very low concentration of impurities in the gas, and therefore the cost can be reduced.

## Claims

1. A method for preparing a calibration gas, comprising the steps of
   - supplying a first zero gas (1) from a first zero gas source (2, 3, 4; 32, 33, 34) with a controlled flow rate (L1),
   - supplying a sample gas from a sample gas source (7) with a controlled flow rate,
   - mixing the sample gas with the first zero gas (1) in a mixing chamber (6) thus forming a first standard gas (10) containing the sample gas in a predetermined concentration,
   - discarding a part of the first standard gas (10) at a first branch (13) with a controlled flow rate (L2), and
   - mixing the remaining first standard gas at a second branch (14) downstream of the first branch (13) with a second zero gas (20) from a second zero gas source (19, 23, 25; 32, 33, 34) with a controlled flow rate (L3) to obtain a diluted, second standard gas,
characterized by

- supplying the sample gas by vaporizing a liquid sample (7) as sample gas source with a controlled evaporation rate,
- discarding a part of the second standard gas at a third branch (15) with a predetermined flow rate (L4), and
- mixing the remaining diluted standard gas at a fourth branch (16) downstream of the third branch (15) with a third zero gas (22) from a third zero gas source (21, 24, 26; 32, 33, 34) with a controlled flow rate (L5), wherein the diluted standard gases are prevented from coming into contact with any parts such as valves and flow controllers, to obtain a calibration gas (18) containing the sample gas in a very low concentration.

2. The method according to claim 1, characterized in that the liquid sample is water or dioctyl phthalate.

3. The method according to claim 1 and/or 2, characterized in that the temperature of the mixing chamber (6) is maintained at a predetermined temperature.

4. The method according to one or several of claims 1 to 3, characterized in that the main tube (12) wherein the standard gases are flowing is maintained at a temperature 20 to 50°C higher than the boiling point of the liquid sample.

5. The method according to one or several of claims 1 to 4, characterized in that the amount of vaporized liquid sample (7) is measured with a microbalance or with an analysis device (38).

6. The method according to one or several of claims 1 to 5, characterized in that the first, second and third zero gases are provided from one gas source line (32, 33, 34).

7. The method according to one or several of claims 1 to 6, characterized in that the zero gas (1, 20, 22) is purified directly before mixing with the sample gas or standard gas, respectively.

8. An apparatus for preparing a calibration gas, comprising
- a first and a second zero gas source (2, 3, 4; 19, 23, 25; 32, 33, 34) for supplying a first zero gas (1) and a second zero gas (20), comprising means (4, 25) for controlling the flow rates (L1, L3) of the first or second zero gases (1, 20), respectively,
- a sample gas source (7) for supplying a sample gas, comprising means for controlling the flow rate of the sample gas,
- a mixing chamber (6) for mixing the sample gas with the first zero gas (1), thus forming a first standard gas (10), and
- a main tube (12) connected with the mixing chamber (6) and having
(a) a first branch (13) comprising controlling means (27) for discarding a part of the first standard gas (10) with a controlled flow rate (L2),
(b) a second branch (14) provided downstream of the first branch (13), at which the remaining first standard gas is mixed with the second zero gas (20) with a controlled flow rate (L3) for obtaining a diluted, second standard gas,
characterized in that
- the mixing chamber (6) contains a sample vessel (8) in which a liquid sample (7) may be vaporized for supplying the sample gas,
- the means for controlling the flow rate of the sample gas are means for controlling the evaporation rate of the liquid sample from the sample vessel (8),
- the main tube (12) is provided with
(a) a third branch (15) comprising controlling means (28) for discarding a part of the diluted second standard gas with a predetermined flow rate (L4), and
(b) a fourth branch (16) provided downstream of the third branch (15), at which the remaining diluted second standard gas is mixed with a third zero gas (22) supplied from a third zero gas source (21, 24, 26; 32, 33, 34) comprising means (26) for controlling the flow rate (L5) of the third zero gas (22), the main tube (12) not being provided with any parts such as valves and flow controllers.

9. The apparatus according to claim 8, characterized in that the sample vessel (8) comprises a variable capillary aperture, preferably of 0.1 to 5 mm, for adjusting the evaporation rate of the liquid sample.

**10.** The apparatus according to claim 8 and/or 9, characterized in that the sample vessel (8) comprises a planar or curved upper surface which has no projection.

**11.** The apparatus according to one or several of claims 8 to 10, characterized in that the tubes are made of electropolished stainless steel or glass, the steel tubes being connected together entirely by welding.

**12.** The apparatus according to one or several of claims 8 to 11, characterized in that control means (39, 40) are provided to control the flow rates of the flow controlling means (4, 25, 26, 27, 28) and the concentration of the calibration gas automatically.

**13.** The apparatus according to one or several of claims 8 to 12, characterized in that the mixing chamber (6) is provided with temperature control means (9) for controlling the evaporation rate of the liquid sample from the sample vessel (8).

**14.** The apparatus according to one or several of claims 8 to 13, characterized in that adsorption reduction means (31) are provided at the main tube (12) for reducing the gas adsorption, preferably means for maintaining the main tube (12) at a temperature 20 to 50 °C higher than the boiling point of the liquid sample (7).

**15.** The apparatus according to one or several of claims 8 to 14, characterized in that impurity removal devices (5, 29, 30) are provided in the zero gas supply lines directly before the mixing chamber (6) or at the branches (14, 16), respectively.

**Patentansprüche**

**1.** Verfahren zur Herstellung von Eichgasen, das folgende Schritte umfaßt:
- Zuführen eines ersten Verdünnungsgases (1) von einer ersten Verdünnungsgasquelle (2, 3, 4; 32, 33, 34) mit kontrolliertem Durchsatz (L1),
- Zuführen eines Probengases von einer Probengasquelle (7) mit einem kontrollierten Durchsatz,
- Mischen des Probengases mit dem ersten Verdünnungsgas (1) in einer Mischkammer (6) unter Erzeugung eines ersten standardisierten Gases (10), welches das Probengas in einer vorgegebenen Konzentration enthält,
- Verwerfen eines Teils des ersten standardisierten Gases (10) an einer ersten Leitungsabzweigung (13) mit einem kontrollierten Durchsatz (L2) und
- Mischen des verbleibenden ersten standardisierten Gases an einer zweiten Leitungsabzweigung (14) stromab von der ersten Leitungsabzweigung (13) mit einem zweiten Verdünnungsgas (20) von einer zweiten Verdünnungsgasquelle (19, 23, 25; 32, 33, 34) mit einem kontrollierten Durchsatz (L3) unter Erhalt eines verdünnten, zweiten standardisierten Gases,

gekennzeichnet durch
- Zuführen des Probengases durch Verdampfen einer flüssigen Probe (7) als Probengasquelle mit einer kontrollierten Verdampfungsgeschwindigkeit,
- Verwerfen eines Teils des zweiten standardisierten Gases an einer dritten Leitungsabzweigung (15) mit einem vorgegebenen Durchsatz (L4) und
- Mischen des verbleibenden verdünnten standardisierten Gases an einer vierten Leitungsabzweigung (16) stromab von der dritten Leitungsabzweigung (15) mit einem dritten Verdünnungsgas (22) von einer dritten Verdünnungsgasquelle (21, 24, 26; 32, 33, 34) mit einem kontrollierten Durchsatz (L5), wobei die verdünnten standardisierten Gase mit keinerlei Teilen wie Ventilen und Durchflußreglern in Kontakt kommen, unter Erhalt eines Eichgases (18), welches das Probengas in einer sehr niedrigen Konzentration enthält.

**2.** Verfahren nach Anspruch 1, dadurch gekennzeichnet, daß die flüssige Probe Wasser oder Dioctylphthalat ist.

**3.** Verfahren nach Anspruch 1 und/oder 2, dadurch gekennzeichnet, daß die Mischkammer (6) auf einer vorgegebenen Temperatur gehalten wird.

**4.** Verfahren nach einem oder mehreren der Ansprüche 1 bis 3, dadurch gekennzeichnet, daß die Hauptleitung (12), in der die standardisierten Gase strömen, auf einer Temperatur gehalten wird, die 20 bis 50 °C höher ist als der Siedepunkt der flüssigen Probe.

5. Verfahren nach einem oder mehreren der Ansprüche 1 bis 4, dadurch gekennzeichnet, daß die Menge der verdampften flüssigen Probe (7) mit einer Mikrowaage oder einer Analysenvorrichtung (38) gemessen wird.

6. Verfahren nach einem oder mehreren der Ansprüche 1 bis 5, dadurch gekennzeichnet, daß das erste Verdünnungsgas, das zweite Verdünnungsgas und das dritte Verdünnungsgas von einer einzigen Gasquellenleitung (32, 33, 34) geliefert werden.

7. Verfahren nach einem oder mehreren der Ansprüche 1 bis 6, dadurch gekennzeichnet, daß das Verdünnungsgas (1, 20, 22) direkt vor dem Mischen mit dem Probengas bzw. einem standardisierten Gas gereinigt wird.

8. Vorrichtung zur Herstellung von Eichgasen, die umfaßt:
   - eine erste und eine zweite Verdünnungsgasquelle (2, 3, 4; 19, 23, 25; 32, 33, 34) zum Zuführen eines ersten Verdünnungsgases (1) und eines zweiten Verdünnungsgases (20), die eine Durchsatzsteuerungseinrichtung (4, 25) zur Steuerung der Durchsätze (L1, L3) des ersten bzw. zweiten Verdünnungsgases (1, 20) aufweisen,
   - eine Probengasquelle (7) zum Zuführen eines Probengases, die eine Durchsatzsteuerungseinrichtung zur Steuerung des Durchsatzes des Probengases aufweist,
   - eine Mischkammer (6) zum Mischen des Probengases mit dem ersten Verdünnungsgas (1) unter Erzeugung eines ersten standardisierten Gases (10) und
   - eine Hauptleitung (12), die mit der Mischkammer (6) verbunden ist und aufweist:
     (a) eine erste Leitungsabzweigung (13) mit einer Durchsatzsteuerungseinrichtung (27) zum Verwerfen eines Teils des ersten standardisierten Gases (10) mit einem kontrollierten Durchsatz (L2) und
     (b) eine zweite Leitungsabzweigung (14), die stromab von der ersten Leitungsabzweigung (13) angeordnet ist und an der das verbleibende erste standardisierte Gas mit dem zweiten Verdünnungsgas (20) mit einem kontrollierten Durchsatz (L3) gemischt wird, wobei ein verdünntes, zweites standardisiertes Gas erhalten wird, dadurch gekennzeichnet, daß
   - die Mischkammer (6) einen Probenbehälter (8) enthält, in dem eine flüssige Probe (7) zur Zuführung des Probengases verdampft werden kann,
   - die Durchsatzsteuerungseinrichtung zur Steuerung des Durchsatzes des Probengases eine Einrichtung zur Steuerung der Verdampfungsgeschwindigkeit der Verdampfung der flüssigen Probe aus dem Probengefäß (8) ist und
   - die Hauptleitung (12) versehen ist mit
     (a) einer dritten Leitungsabzweigung (15) mit einer Durchsatzsteuerungseinrichtung (28) zum Verwerfen eines Teils des verdünnten zweiten standardisierten Gases mit einem vorgesetzten Durchsatz (L4) und
     (b) einer vierten Leitungsabzweigung (16), die stromab der dritten Leitungsabzweigung (15) vorgesehen ist und bei der das verbleibende verdünnte zweite standardisierte Gas mit einem dritten Verdünnungsgas (22) gemischt wird, das von einer dritten Verdünnungsgasquelle (21, 24, 26; 32, 33, 34) geliefert wird, die eine Durchsatzsteuerungseinrichtung (26) zur Steuerung des Durchsatzes (L5) des dritten Verdünnungsgases (22) aufweist,
   wobei die Hauptleitung (12) keinerlei Teile wie Ventile und Durchflußregler aufweist.

9. Vorrichtung nach Anspruch 8, dadurch gekennzeichnet, daß der Probenbehälter (8) eine veränderliche kapillare Öffnung von vorzugsweise 0,1 bis 5 mm Durchmesser zur Einstellung der Verdampfungsgeschwindigkeit der flüssigen Probe aufweist.

10. Vorrichtung nach Anspruch 8 und/oder 9, dadurch gekennzeichnet, daß der Probenbehälter (8) eine ebene oder gekrümmte obere Oberfläche aufweist, die keine Vorsprünge besitzt.

11. Vorrichtung nach einem oder mehreren der Ansprüche 8 bis 10, dadurch gekennzeichnet, daß die Leitungen aus elektropoliertem rostfreiem Stahl oder Glas bestehen, wobei Stahlrohre vollständig durch Schweißen miteinander verbunden sind.

12. Vorrichtung nach einem oder mehreren der Ansprüche 8 bis 11, dadurch gekennzeichnet, daß eine Steuereinrichtung (39, 40) zur automatischen Steuerung der Durchsätze der Durchsatzsteuerungseinrichtungen (4, 25, 26, 27, 28) und der Konzentration des Eichgases vorgesehen ist.

13. Vorrichtung nach einem oder mehreren der Ansprüche 8 bis 12, dadurch gekennzeichnet, daß die Mischkammer (6) eine Temperaturregeleinrichtung (9) zur Steuerung der Verdampfungsgeschwindigkeit der flüssigen Probe aus dem Probenbehälter (8) aufweist.

14. Vorrichtung nach einem oder mehreren der Ansprüche 8 bis 13, dadurch gekennzeichnet, daß eine Einrichtung (31) zur Verringerung der Adsorption an der Hauptleitung zur Verringerung der Gasadsorption vorgesehen ist, vorzugsweise eine Einrichtung, mit der die Hauptleitung (12) auf einer Temperatur gehalten werden kann, die 20 bis 50 °C höher ist als der Siedepunkt der flüssigen Probe (7).

15. Vorrichtung nach einem oder mehreren der Ansprüche 8 bis 14, dadurch gekennzeichnet, daß Einrichtungen (5, 29, 30) zur Abtrennung von Verunreinigungen in den Leitungen zur Zuführung des Verdünnungsgases direkt vor der Mischkammer (6) bzw. an den Leitungsabzweigungen (14, 16) vorgesehen sind.

**Revendications**

1. Procédé de préparation d'un gaz d'étalonnage, comprenant les étapes suivantes :
   - la transmission d'un premier gaz de zéro (1) à partir d'une source de gaz de zéro (2, 3, 4 ; 32, 33, 34) ayant un débit réglé (L1),
   - la transmission d'un gaz échantillon d'une source de gaz échantillon (7) avec un débit réglé,
   - le mélange du gaz échantillon avec le premier gaz de zéro (1) dans une chambre de mélange (6) avec formation d'un premier gaz de référence (10) contenant le gaz échantillon à une concentration prédéterminée,
   - l'évacuation d'une partie du premier gaz de référence (10) vers une première dérivation (13) avec un débit réglé (L2), et
   - le mélange du premier gaz de référence restant dans une seconde dérivation (14) en aval de la première dérivation (13) avec un second gaz de zéro (20) provenant d'une seconde source de gaz de zéro (19, 23, 25 ; 32, 33, 34) avec un débit réglé (L3) pour l'obtention d'un second gaz de référence dilué,
   
   caractérisé par
   - la transmission du gaz échantillon par vaporisation d'un échantillon liquide (7) comme source de gaz échantillon avec une vitesse réglée d'évaporation,
   - l'évacuation d'une partie du second gaz de référence dans une troisième dérivation (15) avec un débit prédéterminé (L4), et

   - le mélange de l'échantillon dilué restant de référence dans une quatrième dérivation (16) en aval de la troisième dérivation (15) avec un troisième gaz de zéro (22) provenant d'une troisième source de gaz de zéro (21, 24, 26 ; 32, 33, 34) avec un débit réglé (L5), dans lequel les gaz dilués de référence ne peuvent pas venir au contact d'une partie quelconque telle que des soupapes ou organes de réglage de débit, afin qu'un gaz d'étalonnage (18) contenant le gaz échantillon à une très faible concentration soit obtenu.

2. Procédé selon la revendication 1, caractérisé en ce que l'échantillon liquide est de l'eau ou du dioctylphtalate.

3. Procédé selon la revendication 1 et/ou 2, caractérisé en ce que la température de la chambre de mélange (6) est maintenue à une valeur prédéterminée.

4. Procédé selon l'une ou plusieurs des revendications 1 à 3, caractérisé en ce que le tube principal (12) dans lequel les gaz de référence circulent est maintenu à une température supérieure de 20 à 50 °C à la température d'ébullition de l'échantillon liquide.

5. Procédé selon l'une ou plusieurs des revendications 1 à 4, caractérisé en ce que la quantité de l'échantillon liquide vaporisé (7) est mesurée avec une microbalance ou avec un dispositif d'analyse (38).

6. Procédé selon l'une ou plusieurs des revendications 1 à 5, caractérisé en ce que le premier, le second et le troisième gaz de zéro proviennent d'une conduite de source de gaz (32, 33, 34).

7. Procédé selon l'une ou plusieurs des revendications 1 à 6, caractérisé en ce que le gaz de zéro (1, 20, 22) est directement purifié avant mélange au gaz d'échantillon ou au gaz de référence respectivement.

8. Appareil de préparation d'un gaz d'étalonnage, comprenant
   - une première et une seconde source de gaz de zéro (2, 3, 4 ; 19, 23, 25 ; 32, 33, 34) destinées à transmettre un premier gaz de zéro (1) et un second gaz de zéro (20), comprenant un dispositif (4, 25) de réglage des débits (L1, L3) du premier ou du second gaz de zéro (1, 20) respectivement,
   - une source de gaz échantillon (7) destinée à transmettre un gaz échantillon et comprenant un dispositif de réglage du débit de l'échantillon gazeux,
- une chambre (6) de mélange du gaz échantillon au premier gaz de zéro (1) avec formation d'un premier gaz de référence (10), et
- un tube principal (12) raccordé à la chambre de mélange (6) et ayant
  (a) une première dérivation (13) comprenant un dispositif de réglage (27) destiné à évacuer une partie du premier gaz de référence (10) avec un débit réglé (L2),
  (b) une seconde dérivation (14) placée en aval de la première dérivation (13) et dans laquelle le premier gaz restant de référence est mélangé au second gaz de zéro (20) avec un débit réglé (L3) pour l'obtention d'un second gaz dilué de référence,

caractérisé en ce que
- la chambre de mélange (6) contient un réservoir d'échantillon (8) dans lequel un échantillon liquide (7) peut être vaporisé pour la transmission de l'échantillon gazeux,
- le dispositif destiné à régler le débit du gaz échantillon étant un dispositif de réglage de la vitesse d'évaporation de l'échantillon liquide du récipient d'échantillon (8),
- le tube principal (12) comporte
  (a) une troisième dérivation (15) comprenant un dispositif (28) de réglage destiné à évacuer une partie du second gaz dilué de référence avec un débit prédéterminé (L4), et
  (b) une quatrième dérivation (16) placée en aval de la troisième dérivation (15) et dans laquelle le second gaz dilué restant de référence est mélangé à un troisième gaz de zéro (22) transmis par une troisième source de gaz de zéro (21, 24, 26 ; 32, 33, 34) comprenant un dispositif (26) de réglage du débit (L5) du troisième gaz de zéro (22),
    le tube principal (12) n'ayant aucune partie telle que des soupapes et des organes de réglage de débit.

9. Appareil selon la revendication 8, caractérisé en ce que le réservoir d'échantillon (8) possède une ouverture capillaire variable, ayant de préférence une dimension de 0,1 à 5 mm, pour l'ajustement de la vitesse d'évaporation de l'échantillon liquide.

10. Appareil selon la revendication 8 et/ou 9, caractérisé en ce que le réservoir d'échantillon (8) comprend une surface supérieure plane ou courbe qui n'a pas de saillie.

11. Appareil selon une ou plusieurs des revendications 8 à 10, caractérisé en ce que les tubes sont formés de verre ou d'acier inoxydable ayant subi un polissage électrolytique, les tubes d'acier étant raccordés entièrement par soudage.

12. Appareil selon une ou plusieurs des revendications 8 à 11, caractérisé en ce qu'un dispositif de commande (39, 40) est destiné à régler les débits des dispositifs de réglage de débit (4, 25, 26, 27, 28) et la concentration du gaz d'étalonnage de manière automatique.

13. Appareil selon une ou plusieurs des revendications 8 à 12, caractérisé en ce que la chambre de mélange (6) comporte un dispositif (9) de réglage de température destiné à régler la vitesse d'évaporation de l'échantillon liquide du réservoir d'échantillon (8).

14. Appareil selon une ou plusieurs des revendications 8 à 13, caractérisé en ce qu'un dispositif (31) de réduction d'adsorption est placé dans le tube principal (12) afin qu'il réduise l'adsorption du gaz, et de préférence un dispositif est destiné à maintenir le tube principal (12) à une température supérieure de 20 à 25 °C à la température d'ébullition de l'échantillon liquide (7).

15. Appareil selon une ou plusieurs des revendications 8 à 14, caractérisé en ce que les dispositifs (5, 29, 30) d'extraction d'impuretés sont incorporés aux conduites de transmission de gaz de zéro directement avant la chambre de mélange (6) ou dans les dérivations (14, 16) respectivement.

# FIG. 1

# FIG. 2

# FIG. 3

# F I G. 4

EXHAUST

EXHAUST

HIGHLY PURIFIED GAS

# FIG. 5

# F I G. 6

FIG. 7

# FIG. 8